# EUROPEAN PATENT APPLICATION

(11) **EP 1 452 484 A1**
(43) Date of publication of application: **01.09.2004**
(21) Application number: 03075514.4
(22) Date of filing: 21.02.2003
(51) Int. Cl.: C01B 15/037, C05F 3/00, C05G 3/02, A61L 2/18, C02F 1/72, C02F 11/14

(54) **Hydrogen peroxide solution**

(71) Applicant: Projectadvies Waalhof B.V., 5612 EK Eindhoven (NL)
(72) Inventor: Bouman, Frans, 3136 JH Vlaardingen (NL)
(74) Representative: Visser-Luirink, Gesina, Dr.

(57) **Abstract**

Described is a hydrogen peroxide solution of hydrogen peroxide in water with at least one stabilizer, suitable as disinfectant and sterilizer, wherein the solution is at least 99% homogeneous, wherein the stabilizer is preferably sorbitol. A large number of methods is described in which this solution can advantageously be applied, such as for instance as disinfectant in the medical and cosmetic sector, in the removal of water from sludge, in climate control and air conditioning systems and in the removal of ammonia from animal manure.

A composition is further described comprising such a hydrogen peroxide solution and one or more contaminating substances. The composition in which nutrient supplements are applied as contaminating substances can advantageously be used in agriculture and horticulture.

## Description

The present invention relates to a hydrogen peroxide solution of hydrogen peroxide in water with at least one stabilizer suitable as disinfectant and sterilizer. The invention further relates to a method for disinfecting products or spaces and diverse other applications, and to a method for preparing such a hydrogen peroxide solution.

The use of a hydrogen peroxide solution to disinfect or sterilize spaces or products, such as for instance in the food industry and packaging industry and in the field of pharmaceutical and cosmetic products, is sufficiently well known and for instance described in European patent application EP 1043273. It is always necessary here to use a stabilizer since a hydrogen peroxide solution deteriorates relatively quickly.

Use has traditionally been made of glycerine, glycerol, silver nitrate and lactic acid as stabilizers. In EP 1043273 the present inventor has described that a polyalcohol, and mannitol in particular, gives reasonably good results, with a deterioration of 0.4% in 14 days.

A hydrogen peroxide solution, particularly in concentrated form with an even greater stability, does however remain very desirable. Surprisingly, it has been found that applying sorbitol as stabilizer makes the hydrogen peroxide solution extremely homogeneous, whereby the hydrogen peroxide solution according to the invention has particular properties. The present invention therefore provides a solution of the type stated in the preamble, characterized in that the solution is at least 99% homogeneous, preferably at least 99.5% homogeneous. This high degree of homogeneity makes it possible that contaminating substances can be added thereto with which this would otherwise not have been possible, since a reaction with the hydrogen peroxide would have taken place.

As stabilizer the hydrogen peroxide solution in particular comprises sorbitol, more in particular D-sorbitol, also known as D-glucitol. More preferably D-sorbitol is applied with a non-toxic value of at least 0.0002%, or even more preferably 0.0001%, and a purity of at least 98%, preferably of at least 98.5%. A wide application in the food industry and pharmaceutical industry hereby becomes possible.

In a particular embodiment the hydrogen peroxide solution according to the present invention comprises 10 - 50% by weight of hydrogen peroxide, more in particular 35 - 50% by weight. The great advantage of such concentrated solutions with sorbitol is that after 100 days a deterioration of only about 0.4% occurs, i.e. 99.6% of the initial concentration of hydrogen peroxide is still present in the solution. Such a hydrogen peroxide solution in sorbitol is also designated AQUA-TEN™.

More preferably the homogeneous hydrogen peroxide solution further comprises at least one organic acid, for instance hexanoic acid, pentanoic acid, citric acid, lactic acid or glycol acid. An organic acid is particularly important if the solution is used in an environment above room temperature, and particularly in the tropics, and in addition also acts as a fungicide.

The stabilizer, and then sorbitol in particular, is preferably present in deficiency in the hydrogen peroxide solution, wherein the weight ratio of hydrogen peroxide and the stabilizer, in particular sorbitol, lies between 5:1 and 50:1, more preferably between 7:1 and 10:1.

In a preferred embodiment the invention further provides a composition containing a hydrogen peroxide solution according to the present invention together with contaminating substances. In this application contaminating substances are understood to mean substances which react with hydrogen peroxide, and in many cases react even violently with the atomic oxygen present in hydrogen peroxide while forming radicals. Examples of such substances are salts and esters of alkali metal and alkaline-earth metal ions, of acid radical ions such as halogenides in the ion form such as for instance chlorine, and other trace elements. Salts of alkali metal and alkaline-earth metal ions such as potassium and calcium, as well as other trace elements such as silicon and selenium can serve as nutrient supplements.

According to a further aspect of the invention, there is provided a method for administering biocide and fertilizer to agricultural or horticultural products in one operation, wherein such a composition containing a hydrogen peroxide solution is applied together with contaminating substances. This root and leaf feed is also designated MULTE RESISTANT™. This is a particularly advantageous application of the stable hydrogen peroxide solution, wherein both biocide in the form of hydrogen peroxide and nutrient supplements such as sodium, potassium and phosphate can be administered simultaneously to agricultural or horticultural products in one operation. The use of standard chemicals in which the nutrient supplements are normally dissolved can be reduced by 15 - 20%, which is a great advantage not only economically but also ecologically.

According to yet another aspect of the invention the hydrogen peroxide solution according to the invention can be advantageously applied in a climate control and air-conditioning system, whereby the atmosphere in a space can be controlled, wherein the climate control and air-conditioning system and the atomizing system themselves comprising such a hydrogen peroxide solution also form an aspect of the invention.

According to yet another aspect of the invention, there is provided a method for disinfecting products or spaces, wherein a hydrogen peroxide solution according to the invention is applied. The solution can be applied particularly advantageously in the removal of the so-called biofilm, a layer of fat in for instance pipes which remains after the transport of water or aqueous solutions through the pipes in question, or on the inside of a car window, where a biofilm layer builds up gradually due to the air circulation. The legionella infection, which is dangerous to humans, is also countered by removing the biofilm. Reduced biofilm formation is here also a significant extra advantage in the above mentioned feeding of crops by means of a homogeneous hydrogen peroxide solution together with nutrient supplements via capillary tubes. This is a great improvement relative to the traditionally applied stabilizer glycerine, which in the presence of lipids results in blockage of the capillary tubes.

The solution according to the invention can also be advantageously applied in the disinfection of swimming water as a substitute for the much used chlorine, which is of particular significance for swimmers with bronchial disorders. The hydrogen peroxide solution can also be advantageously applied in the protection and storage of foodstuffs due to the strongly reduced effect on pathogen formation of harmful microorganisms. Reductions have been measured here from a pathogen count higher than 1,000,000 reduced to a pathogen count lower than 3,000 to 4,000. The hydrogen peroxide solution according to the invention can further be used as a disinfecting shower after work in for instance the cattle farming sector.

The stable and non-toxic homogeneous hydrogen peroxide solution preferably having sorbitol as stabilizer can also be advantageously applied according to the invention in the field of personal hygiene, such as for instance in oral and skin care, in the treatment of wounds which do not close easily or warts, and in deodorants. The application of this hydrogen peroxide solution in the total medical sector, such as in disinfecting of operating equipment and the like, can also be envisaged.

The application of the hydrogen peroxide solution according to the invention in keeping clean equipment wherein foodstuffs are processed, cleaned, packaged and stored, such as processing machines, refrigerators, freezers, breadbins and the like, also falls within the scope of the invention.

According to another aspect of the invention, there is provided a method for removing ammonia from animal manure, wherein a hydrogen peroxide solution according to the present invention is applied. In this manner ammonia can be completely removed, wherein the above described problem with the use of glycerine in the presence of lipids which are found in the plant-like substances in manure is also avoided in this application. The most important fields of application are formed by the atomizing of a small quantity of the solution over chicken and pig manure, wherein the released ammonia gas is converted into ammonium salt. If the ventilation in a poultry farm fails, it will be possible to prevent the mass death of the chickens, which occurs within 5 minutes, by applying the hydrogen peroxide solution in atomized form.

According to another important aspect of the invention, there is provided a method for removing water from aqueous sludge, wherein a hydrogen peroxide solution according to the present invention is applied. The removal of water from sewage sludge is important for the storage of sludge, wherein separation is arranged between the aqueous phase and the solids in this same sludge. The hydrogen peroxide solution according to the invention breaks the binding between the aqueous phase and the solids usually bound by polymers. The degree of water removal which can be achieved is related to the type of sludge.

Finally, as special application, the use can be mentioned of the hydrogen peroxide solution according to the invention in separating techniques, for instance of animal manure, wherein particles floating in a liquid medium are expelled and chemically converted into other elements. For this purpose the solution is mixed with the animal manure, whereafter the mixture undergoes a further treatment and is carried along a set of electrodes, wherein in addition to the separations a large stream of gas bubbles is also produced at the electrodes by electrochemistry.

The invention also provides a method for preparing a hydrogen peroxide solution according to the invention, wherein the stabilizer is mixed with the hydrogen peroxide solution in a mixer, wherein the height is greater than the diameter, with an extracting means in the top of the mixer and an outlet to the outside.

An example of safe manufacture of the hydrogen peroxide solution takes place in a round mixer which is provided with a propeller mixing gear with a maximum rotational speed of 900 rotations/min. The ratio of the mixer (height : diameter) must be 2:1. At said height there results a heat of reaction of a maximum of 1°C. It is recommended to make a mixing tank no larger than 1250 litres. The outlets are preferably all provided with a static, stainless steel mixer of a minimum of 3 revolutions. The use of ball valves is recommended in respect of the low turbulence of these valves.

In the top of the mixer an extracting means must preferably be placed with an outlet outside the mixing area. To prevent unintended and unexpected reactions, it is necessary to provide the mixer with a stainless steel annular conduit which is connected to a storage tank filled with an iron compound, for instance an iron chelate. Such a reaction can be weakened by adding iron chelate when the reaction occurs.

It will be apparent that the solution has a very wide field of application, which is in fact only possible due to the prolonged action of the solution according to the invention. If the action were less sustained, treatment would have to be carried out repeatedly with short intervals, or new solutions would have to be introduced repeatedly into a system.

Preferably taken as starting point for the solution according to the invention is a stabilized 50% solution of hydrogen peroxide, at least almost pure sorbitol and one or more organic acids. As already stated above, the action of the hydrogen peroxide solution can be further improved by adding an organic acid to the solution, which will preferably be in a quantity of 1-5% by weight of organic acid.

When the hydrogen peroxide solution is atomized or sprayed, the organic acid can for instance be pentanoic acid, hexanoic acid or glycol acid, wherein a solution according to the invention with glycol acid will be used mainly to preserve diverse foodstuffs. Such a solution destroys all possible contaminants, germs, fungi, bacteria by combustion without adding its own odour or taste to the product and also without discolouring the product. In the application as nutriment for plants in a combination with nutrient supplements, atomizing is the appropriate method because of the danger of undesired reactions when droplets of hydrogen peroxide solution are exposed to sunlight.

The wide utility of the hydrogen peroxide solution according to the invention is clearly shown when the application of the stabilizer sorbitol, with which hydrogen peroxide forms a homogeneous solution, is compared to the application of other stabilizers, see the table appended below.

| H₂O₂ with | action | utility (%) |
|---|---|---|
| glycerine | not in manure and plants | 75 |
| glycerol | not in manure, plants and legionella | 65 |
| silver nitrate | toxic | 20 |
| lactic acid | only at low concentrations | 15 |
| mannitol | not suitable with contaminating substances | 70 |
| sorbitol | | 99 |

It will be apparent to the skilled person that the inventive concept allows a wide application of the homogeneous hydrogen peroxide solution as described here, and is certainly not limited to the examples given in this description.

## Claims

1. Hydrogen peroxide solution of hydrogen peroxide in water with at least one stabilizer, suitable as disinfectant and sterilizer, **characterized in that** the solution is at least 99% homogeneous.

2. Hydrogen peroxide solution according to claim 1, **characterized in that** the solution is at least 99.5% homogeneous.

3. Hydrogen peroxide solution according to claim 1 or 2, **characterized in that** the solution comprises sorbitol as stabilizer.

4. Hydrogen peroxide solution according to one or more of the foregoing claims, **characterized in that** sorbitol is D-sorbitol.

5. Hydrogen peroxide solution according to one or more of the foregoing claims, **characterized in that** the solution comprises 10 - 50% by weight of hydrogen peroxide,.

6. Hydrogen peroxide solution according to claim 5, **characterized in that** the solution comprises 35 - 50% by weight of hydrogen peroxide.

7. Hydrogen peroxide solution according to one or more of the foregoing claims, **characterized in that** the solution further comprises at least one organic acid.

8. Hydrogen peroxide solution according to claim 7, **characterized in that** the organic acid is hexanoic acid, pentanoic acid, citric acid, lactic acid or glycol acid.

9. Hydrogen peroxide solution according to one or more of the foregoing claims, **characterized in that** the weight ratio of hydrogen peroxide and sorbitol lies between 5:1 and 50:1.

10. Hydrogen peroxide solution according to claim 9, **characterized in that** the weight ratio of hydrogen peroxide and sorbitol lies between 7:1 and 10:1.

11. Composition comprising a hydrogen peroxide solution according to one or more of the claims 1-10 and one or more contaminating substances.

12. Composition according to claim 11, wherein the contaminating substances are nutrient supplements.

13. Composition according to claim 12, wherein the nutrient supplements are chosen from the group consisting of salts or esters of sodium, potassium and phosphate.

14. Climate control and air-conditioning system comprising a hydrogen peroxide solution according to one or more of the claims 1-10.

15. Atomizing system comprising a hydrogen peroxide solution according to one or more of the claims 1-10.

16. Method for disinfecting products or spaces, **characterized in that** a hydrogen peroxide solution according to one or more of the claims 1-10 is applied for the disinfecting.

17. Method according to claim 14, **characterized in that** a biofilm is removed during the disinfecting.

18. Method for removing ammonia from animal manure, **characterized in that** a hydrogen peroxide solution according to one or more of the claims 1-10 is applied.

19. Method for removing water from aqueous sludge, **characterized in that** a hydrogen peroxide solution according to one or more of the claims 1-10 is applied.

20. Method for administering biocide and fertilizer to agricultural or horticultural products, **characterized in that** a composition according to claim 12 or 13 is applied.

21. Method for controlling the atmosphere in a space, **characterized in that** the climate control and air-conditioning system according to claim 14 is applied.

22. Method for preparing a hydrogen peroxide solution according to one or more of the claims 1-10, wherein the stabilizer is mixed with the hydrogen peroxide solution in a mixer, wherein the height is greater than the diameter, with an extracting means in the top of the mixer and an outlet to the outside.
